# EUROPEAN PATENT SPECIFICATION

(11) **EP 1 529 554 B1**
(45) Date of publication and mention of the grant of the patent: **02.08.2006**
(21) Application number: 03025416.3
(22) Date of filing: 05.11.2003
(51) Int. Cl.: A61N 5/10

(54) **Radioactive radiation source for ophthalmic brachytherapy**
Radioaktive Strahlungsquelle zur ophtalmischen Brachytherapie
Source radioctive de rayonnement pour la brachythérapie ophtalmique

(43) Date of publication of application: 11.05.2005
(73) Proprietor: NeoVista, Inc., Duluth, Georgia 30097 (US)
(72) Inventor: Behrmann, Sabine, 38110 Braunschweig (DE); Fritz, Eberhard Dr., 38110 Braunschweig (DE)
(74) Representative: Schwabe - Sandmair - Marx

(56) References cited:
- WO-A-01/43826
- US-A1- 2001 027 261
- US-B1- 6 387 035

## Description

The present invention relates to a radioactive radiation source for brachytherapy, and especially a radiation source for localized delivery of radiation in surgical procedures, particularly ophthalmic procedures.

### Background of the invention

Current therapeutic approaches to ophthalmic diseases, and especially macula degeneration may to a certain extent involve a radiation treatment of the inflicted parts of the macula. More specifically, the current state of the art brachytherapy for treatment of localized lesions, especially tumors and macula degeneration employs radioactive sealed radiation sources. The term "sealed" means that radioisotopes incorporated into a device are integral with the device and cannot be dislodged or released from the host material of the device in the environment of usage. A typical sealed radiation source includes a radiation source encapsulated within an impermeable, biocompatible capsule material, for example titanium, which is designed to prevent any leaching or a release of the radioisotope. The source is typically about 4.5 mm long and has a diameter of about 0.8 mm and is implanted individually at a treatment side within or around a lesion by using hollow delivery needles. These sources suffer from the disadvantage that they provide a homogeneous coaxial radiation pattern over their entire circumference resulting in undesirable irradiation of surrounding healthy tissue.

In the treatment of intraocular tumors in another approach hemispherical ophthalmic plaques are used as sources, incorporating a radioactive material are sewn directly to the eyeball to provide a radiation dose to the intraocular tumor on the concave side of the plaque. These plaques comprise typically a thin film of Ru-106 encapsulated within two sheets of silver, the sheet on the concave side being approximately 0.1 mm thick and the sheet on the convex side being approximately 0.7 mm thick. Accordingly, these sources provide partial shielding of healthy tissue. Greater sheet thickness provides additional radiation shielding but adds to the thickness of the plaque, which increases discomfort to the patient.

In addition, US patent 6 030 333 discloses an implantable radiotherapy device or radiation source for delivering a predetermined dose of radiation in a predetermined pattern, which device includes a biocompatible template and one or more radiation sources which are incorporated directly into at least a portion of the template preferably by ion implantation methods. The emitted radiation pattern and dose is exclusively determined by the pattern of implanting the radiation emitting material into the device or carrier. These devices are disadvantageous in that the radioactive material is irrevocably affixed to the entire device such that finally the entire device needs be deposited after final use thereof.

Other approaches have been used for a non-permanent treatment. For example US patent 6 443 881 discloses a method and an apparatus for use in ocular or ophthalmic brachytherapy. The device comprises a handle, an applicator coupled with said handle, and adapted to receive a source of radiation. The typically concave shaped applicator is movable between a radiation shielding position and a position wherein radiation is allowed to reach the diseased area. A shield receives the applicator so as to shield the radiation source during insertion and positioning proximal to the treatment side. Some sort of shielding may further be provided to prevent emission of radiation in direction towards other tissues than the retina to be treated. In the treatment position, the radiation is directed outwardly through an open area opposing the application site.

A comparable device is disclosed in US-B1-6 285 735. This document discloses an applicator system for applying a dose of x-ray radiation across a predefined contour of a body to treat a predefined volume of tissue. The adapter includes an applicator endcap including a substantially planar or convex treatment surface for engating and conforming a tissue cavity to the desired shape in order to permit the volume of adjacent tissue to be treated with a predefined dose of radiation. The applicator may include a shank having a substantially conical void region to form the punctual radiation beam. The shank may further include an endcap of an appropriate form to facilitate the application of radiation to a surface area and to modulate the emitted radiation uniformly over the surface. The endcap is preferably formed from a biocompatible acrylic material and fixed to the shank using an epoxy resin. The endcap is directly in contact with the tissue to be treated and is not in contact with the radiation source or probe as defined in the document. Just in contrast, the endcap forms part of the applicator device, not the radiation source itself.

US patent US-B1-6 387 035 discloses a brachytherapy source comprising the features of the preamble of claim 1.

Published US patent application No. US 2002/01 15 902 discloses a surgical device for localized delivery of beta-radiation in surgical procedures, particularly ophthalmic procedures, which device includes a canula with a beta-therapy emitting material at the distal end of the canula. The device may further comprise a shield at the distal end of the canula for shielding the beta-radiotherapy emitting material. This shield at the distal end of the canula may be a thin wall of metal such as stainless steel, or maybe a thin wall of polymer, plastic or similar material. The shield may be retractable to expose the beta-radiotherapy emitting material for treatment. There is, despite of the considerable efforts in the art, no possibility of effectively shielding the surrounding tissue, when homogeneously irradiating the desired site of treatment with a much smaller source.

### Summary of the invention

These and other objects can be solved and the drawbacks of the prior art can be overcome by a radioactive radiation source for brachytherapy having an elongated radiation emitting element (1) within an elongated means for containment (2) such that the longitudinal axis of the radiation emitting element and the longitudinal axis of said means for containment are aligned, said means for containment comprising a shielding section (3) and a radiation transition section (4),
- said shielding section (3) covering said radiation emitting element at least partially to substantially attenuate any radiation emitted in the direction of said shielding section,
- said radiation transition section (4) extending substantially along the longitudinal axis of the means for containment and comprising a shielding material (5),
- which shielding material (5) is so adapted as to attenuate the radiation emitted from said radiation emitting element such that, in a plane at a pre-selected distance from the radiation source, a substantially uniform radiation dose is received over a target area (6) having a length substantially larger than the longitudinal axis of the elongated means for containment, and preferably a diameter substantially larger than the diameter of the means for containment.

The shielding material (5) may be adapted to attenuation of the radiation by varying its thickness, density and/or composition. In general, said shielding material may be adapted by varying its shielding properties.

Other preferred embodiments are as set out in the appending claim.

### Description of the Drawings

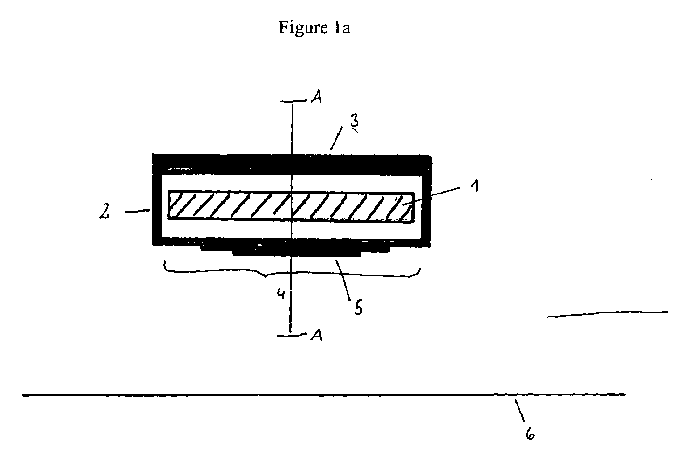

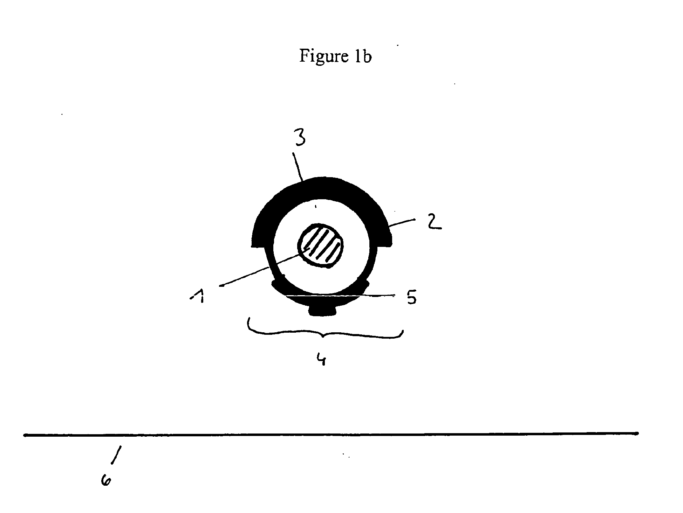

Fig. 1a and b show an exemplary embodiment of the radioactive radiation source of the invention. In Fig. 1a a longitudinal cross-section is shown, whereas Fig. 1b shows a cross-section along line A-A in Fig. 1a. In this embodiment of Fig. 1a radiation emitting element (1) is provided in form of a wire. The means for containment (2) integrally comprises a thick shielding section (3) and a radiation transition section (4) on the second halfcircle of the cylindrical means for containment (2). The shielding section (4) comprises the shielding material (5) adapted by varying its thickness. Specifically, the thickness of material (5) is largest in those areas of the means for containment (2) closest to the target area (6), whereas on more laterally and terminally located parts it has a smaller degree of thickness.

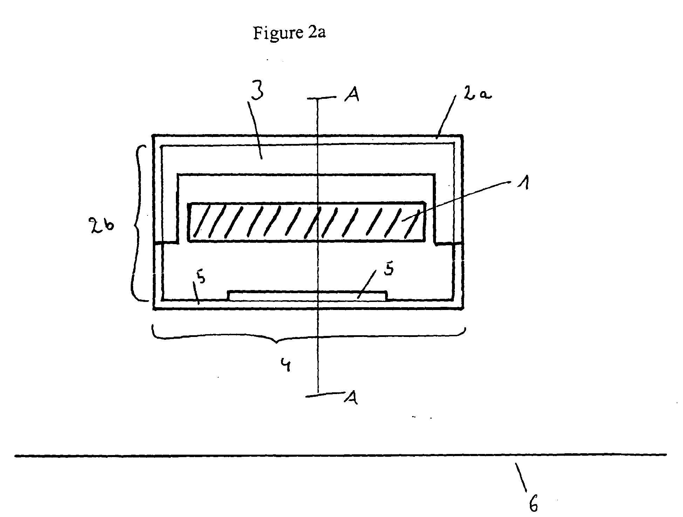

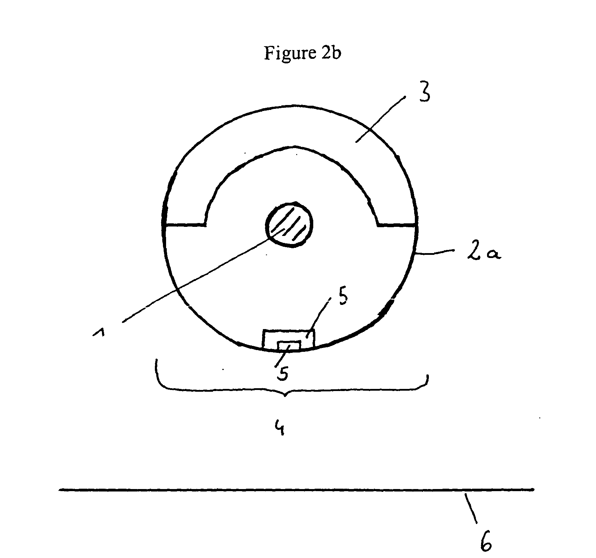

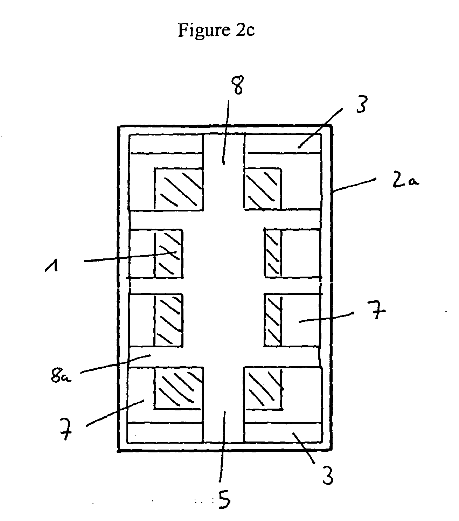

Fig. 2a-c show an alternative embodiment of the radioactive radiation source of the invention. Fig. 2a again shows a longitudinal cross-section, whereas Fig. 2b shows the cross-section along line A-A in Fig. 2a. Fig. 2c shows a bottom view of the source after the first capsule (2a) of the means for containment has been peeled off.

Specifically, in this case the means for containment (2) comprises a first capsule (2a) in form of a first sealed metallic cylinder having endplugs. A second metallic cylinder is provided within said first cylinder. The first or upper half circle of the second cylinder within the first cylinder forms a shielding section (3) and the second half circle forms a radiation transition section (4). The shielding material (5) in the radiation transition section (4) is adapted by varying the wall thickness of the second cylinder. In this embodiment, as can be seen in Fig. 2c 8 recesses (7) are provided parallel to a central bar forming cross bridge (8). The 4 recesses on each side are again separated from each other by bars (8) extending in cirumferrential direction of the second cylinder. Bar (8) is aligned with the central axis of both the first metallic cylinder (2a) and the radiation emitting element (1) within the same. Typically bar (8) is arranged directly opposite the target area (6) such that radiation emitted from the radiation emitting element (1) in a direction normal to the target area will be more strongly attenuated as compared to radiation emitted in a slightly tilted direction through the recesses (7). Thereby, a homogenous radiation can be achieved.

### Detailed Description of the Invention

The radioactive radiation source of the invention is suitable for brachytherapy and especially for ocular or ophthalmic brachytherapy such as in the treatment of macula degeneration, preferably age related macula degeneration (AMD). The radiation source of the invention comprises a radiation source for brachytherapy having an elongated radiation emitting element (1) within an elongated means for containment (2) preferably arranged such that the longitudinal axis of the radiation emitting element and the longitudinal axis of said means for containment are aligned. Said means for containment comprises a shielding section (3) and a radiation transition section (4). Said shielding section (3) covers said radiation emitting element at least partially to substantially attenuate any radiation emitted in the direction of said shielding section. Preferably the shielding section covers the element to about 30-90 %, preferably 40-70 % and more preferably 50-60 %.

Said radiation transition section (4) extends substantially along the longitudinal axis of the means for containment and comprises a shielding material (5), which shielding material (5) is so adapted as to attenuate the radiation emitted from said radiation emitting element such that, in a plane at a pre-selected distance from the radiation source, a substantially uniform radiation dose (deviations less than 30 %, preferably less than 20 %, more preferably less than 10 %) is received over a target area (6) having a length substantially larger than the longitudinal axis of the elongated means for containment, and, preferably a diameter substantially larger than the diameter of the means for containment. The term "substantially" as used in this context generally means at least 50 % enlargement preferably 100 to 200 % enlargement in the intended dimension.

As used herein the term "elongated" means any shape of the radiation emitting element having one axis substantially larger than the other two axes. Preferably the aspect ratio of the elongated radiation emitting element (ratio of longitudinal axis to diameter) is ≥ 2:1, more preferably 5:1 to 25:1 and most preferably 8:1 to 17:1, especially 10:1. In general, the radiation emitting element may have any cross section, provided this cross section does not prevent its movement within a catheter or any other delivery device. Preferably, the cross section is circular, ellipsoid or polyhedric, circular cross-sections being preferred. In case of non-circular cross-sections the above aspect ratio is determined using the largest diameter in a plane perpendicular to the longitudinal axis.

The radiation emitting element may generally adopt any suitable form, as long as the form is elongated and does not interfere with its intended purpose. Typically it will be a single solid or hollow body, but may also be composed of several individual elements arranged in series within the means for containment to form the elongated shape of the element. The element preferably is a single body, which may itself be hollow or solid. More preferably the radiation emitting element is a cylindrical wire, optionally wound in form of a coil, or a tube. Alternatively, if consisting of several elements, these may be spheres or ellipsoids arranged in series.

The elongated radiation emitting element is sealingly enclosed within the means for containment. This means for containment (2) has an elongated shape, its longitudinal axis being typically being aligned with and preferably being arranged in parallel to the longitudinal axis of the radiation emitting element. An arrangement "in parallel" as referred to herein does not exclude a slight tilting of the respective longitudinal axes towards each other e. g. up to 20°, preferably to 10°. This is as long as the elongation is substantially within the same direction. Preferably the means for containment in its dimensions matches the outer dimensions of the elongated radiation emitting element, such that the latter tightly fits within the means for containment.

The outer dimensions of the means for containment or cover sleeve, where applicable, will typically define the dimensions of the radioactive radiation source as well. The radiation source itself may generally have any length as considered appropriate by the skilled worker for the chosen side of treatment. Typically it will have a length in the range of ≤1 to 25 mm, preferably 1 to 15 mm, more preferably 2 to 10 mm and most preferably 2 to 5 mm. Likewise, the radiation source will typically have a diameter in the range of 0.1 to 2.0 mm, preferably 0.6 to 1.2 mm, most preferably 0.8 to 1.2 mm.

The radiation emitting element (1) typically has a compatible outer diameter in the range of 0.1 to 1 mm, preferably 0.2 to 0.8 mm. More preferably the radiation emitting element has an outer diameter in the range of 0.1 to 0.5 mm and most preferably 0.3 to 0.4 mm. The inner diameter of the means for containment (2) is chosen appropriately to receive the element (1) therein. The above-referenced dimension of the radiation source of the invention typically relate to the means for containment thereof. The radiation source of the invention may, however, also be affixed to a catheter or an applicator of appropriate length for advancing the radiation source to the desired site of treatment. Similarly the source may be brought in position by using an applicator or a catheter using the conventional afterloading technology. In a preferred embodiment the means for containment forms an integral part of the catheter/applicator at its tip as is e. g. disclosed in US laid open publiction 2002/0115902 and the radiation emitting element is fixed therein.

In an alternative embodiment the shielding section and the radiation transition section may, optionally together with a cover sleeve, be inbuilt and integral to the applicator tip. A conventional seed including a radiation emitting element sealingly enclosed in a capsule may then be moved by afterloading into the tip of the applicator to form the final radiation source of the invention.

The means for containment may in addition to the elongated radiation emitting element also include a radioopaque marker to allow monitoring of the advance of the radiation source in use. Such radioopaque marker may be provided in the central core within a hollow coil-shaped or hollow cylindrical radiation emitting element, and/or may be provided as separate body on one or both sides of the radiation emitting element or interspersed between e. g. several spheres, forming said radiation emitting element. Other embodiments of including a radioopaque marker within the means for containment comprising the radiation emitting element are well known to the skilled worker. These are encompassed by the present invention.

The means for containment comprises a shielding section (3) and a radiation transition section (4). These two sections may together form the means for containment or sections thereof or may be provided as separate and distinct sections on the inside or outside of a first layer or capsule (2a), which layer or capsule sealingly encloses the radiation emitting element to provide the containment function. As an example, the first capsule (2a) may be formed in form of a hollow cylinder having endplugs, which capsule sealingly encloses the radiation emitting element. Within or around, but preferably within capsule (2a) there can be provided a second cylinder comprising and/or forming the shielding section (3) and the radiation transition section (4) the latter being e. g. provided by varying wall thickness of the second cylinder on one side thereof. Providing these sections within the first capsule has the advantage that any sharp edges on the outer side of the source can be prevented which may cause injuries during insertion or removal. Alternatively a cover sleeve may be provided as discussed below.

In another embodiment the shielding section (3) and the radiation transition section (4) are integral parts of the means for containment (2). Again the radiation transition section (4) may be provided e. g. by varying and especially decreasing the wall thickness of the means for containment in a pre-determined section thereof.

The shielding section (3) covers the radiation emitting element at least partially to substantially attenuate any radiation emitted in the direction of said shielding section. As used herein the term "substantially attenuate" means attenuation of radiation in the shielded direction to desirably low levels to prevent damage of surrounding tissue at the site to be treated and preventing undesired exposure of medical personal. Specifically, attenuation is preferably achieved to decrease the radiation by more than 90 %, preferably more than 99 % and most preferably 100 % of the emitted radiation in said direction.

The radiation transition section (4) forms a second part of the means for containment (2). This section extends substantially along the longitudinal axis of the means for containment. With the term "extending substantially along" as used herein it is meant that the radiation transition section is aligned over the longitudinal axis of the means for containment, preferably aligned centrally over the longitudinal axis of the means for containment and corresponds in its length at least to a larger portion of the length of said longitudinal axis of the radiation emitting element comprised within the means for containment and preferably of the means for containment itself. Typically, the radiation transition section will extend itself over and along of more than 50 % of the longitudinal axis of the means for containment, more preferably 60 to 100 % thereof and most preferably 75 to 95 % thereof.

The radiation transition section (4) is located on the side or face of the radiation source of the invention (its means for containment) opposing or intended to oppose the target area to be irradiated.

The radiation transition section (4) comprises a shielding material (5). This shielding material (5) is so adapted as to attenuate the radiation emitted from the radiation emitting element in such a manner that, in a plane at a preselected distance from the radiation source, a substantially uniform radiation dose is received over a target area (6), having a diameter substantially larger than the diameter of the means for containment and a length substantially larger than the longitudinal axis of the elongated means for containment.

Adaption of the shielding material (5) occurs by adapting its shielding properties. This may be achieved through varying its thickness, density and/or composition. In a preferred embodiment the shielding material (5) is adapted by varying its thickness. In this embodiment the adaption may specifically be achieved by providing the shielding material in a greater thickness on such portions of the radiation transition section (4) closer to the target area (6), than on those further away from the same.

Taking into account that the emitted radioactive radiation suitable for brachytherapy is effectively shielded even in air, let alone a body fluid or tissue, any travel distance of the radiation beam through (i) means of containment, (ii) fluid or air, and (iii) tissue will effectively decrease the radiation dose received in the preselected target area. Any radiation emitted in a direction normal to the plane of the target area (i. e. on the part of the source closest thereto) will have to travel the shortest distance and will thus experience the least attenuation. Any radiation emitted in a direction tilted with regard to the normal direction will travel longer and will thus experience a higher degree of attenuation, the degree of attenuation depending on the travel distance and hence the angle of tilting as well as the medium through which the radiation passes.

To allow for homogeneous radiation doses being achieved in a target area larger than the dimensions of the source itself, radiation emitted in tilted directions need to be used as well. These are, however, prone to deliver a smaller radiation dose at the target area. The present invention provides the shielding material to modulate (by appropriate attenuation of) the emitted radiation especially the radiation emitted in normal direction, to cope with this difference and to finally achieve a homogeneous radiation dose in the preselected target area. Thereby very small radiation sources can be used to effectively and homogeneously irradiate a larger target area.

In an especially preferred embodiment the shielding section (3) may cover the radiation emitting element (1) over the full length of its longitudinal axis on at least one side of the element. Accordingly, substantial emission of radiation to this side of the radiation source can be prevented. The radiation transition section (4) is then preferably located on the side of the radiation emitting element opposite to the shielding section (3).

In a preferred embodiment the shielding material (5) may advantageously be provided with higher shielding capacity (e. g. in a greater thickness) centrally over and along the longitudinal axis of the radiation emitting element on that part closest to the target area. Besides this central part a shielding material of lower shielding capacity (e. g. of smaller thickness) is provided. This can be either throughout the full length of the central part or throughout a partial length thereof. Preferably, the shielding material of smaller thickness is provided on both sides of the central part either over the full length of the same or towards the ends of the radiation emitting element, leaving an area of higher shielding capacity (e. g. larger thickness) towards the central part of the radiation emitting element.

The most preferred embodiment, the shielding material (5) may comprise recesses (7), in particular recessed windows (no shielding material), on such portions of the radiation transition section (4) further away from the target area (6). The shielding material (5) is in this case again provided centrally over or along the longitudinal axis of the radiation emitting element, the one or more recesses being provided on either or both sides thereof. Preferably arrangement of more than one (2-10, preferably 4-8) recesses is symmetrical to the central axis of shielding material (5).

The shielding material (5) may also be adapted by varying its shielding effect through varying its density and/or composition. In this case a material with a high shielding effect may be used on such portions of the radiation transition section (4) closer to the target area (6), while on such portions of the radiation transition section (4) further from the target area (6) a material with a lower shielding effect may be used. The composition of the shielding material may e. g. be changed throughout the shielding material by techniques such as iron bombardment, sputtering and so on.

More specifically, in case of a metallic shielding material, its composition may be changed by iron bombardment with a heavier metallic material to achieve the desired change in composition. For example the more transmitting sections of the radiation transmitting section (4) may be masked during iron bombardment and all other parts may be bombarded with a heavier material to increase their attenuation factor to the desired extent.

In a preferred embodiment, the shielding material (5) of the radiation transition action (4) is so adapted that the radiation emitted through said radiation transition section (4) is incident on a target area of substantially planar circular form to provide a substantially uniform radiation dose in said plane.

According the embodiment shown in Fig. 1 the shielding section (3) and the radiation transition section (4) form integral parts of the means for containment (2).

Alternatively, as shown in Fig. 2 the means for containment (2) may comprise a first layer or capsule (2a), which layer or capsule sealingly encloses the radiation emitting element (1) and further comprises a shielding section (3) and a radiation transition section (4), which are provided separately from the first layer or capsule (2a) on the outside (not shown) or inside thereof. Preferably, the shielding section and the radiation section are provided on the inside of the layer or capsule (2a) as shown in Fig. 2. In this for example, the means for containment (2) comprises a first capsule (2a) in cylindrical form, which capsule is provided with endcaps to sealingly enclose the radiation emitting element (1). The shielding section (3) and the radiation transition section (4) may then be provided in form of a second cylinder. The radiation transition section (4) is provided on the second cylinder by appropriately adapting the wall thickness of the second cylinder in one halfcircle thereof or by changing its composition through iron bombardment in appropriate manner as disclosed above. The second cylinder may preferably be affixed to the first capsule by any appropriate means, e. g. by welding or use of an adhesive.

The emitting element according to the present invention may comprise any appropriate radioactive radiation emitting nuclide as known suitable for medical purposes. IN one preferred embodiment the radiation emitting element is a β-radiation emitting element. Preferably nuclides are chosen having a maximum particle energy of β-radiation of at least 500 keV and preferably no photon energy for γ-radiation, if possible. Alternatively x-ray or soft γ emitting nuclides with photon energies between 20 keV and 200 keV, more preferably 20 keV and 100 keV may be used. These radioactive materials are soft emitters which are most desirably used in treatment of biological materials and tissue, due to their short attenuation distance. β-source electrons of these energies typically only penetrate only 1 to 10 mm into human tissues. They are easily shielded by even plastic materials. Accordingly, the damage to neighbouring tissues surrounding the site to be treated can be minimized.

In a preferred embodiment the radiation emitting element comprises a nuclide selected from the group consisting of Y-90, Sr-90/Y-90, Tm-170, P-32, Cl-36, Ce-144, Pr-144, Tb-160, Ta-182, Tl-204, Sn-123, Re-188, Ir-192 and Se-75. Most preferably, the radiation emitting element comprises one of the nuclides Y-90, Tl-204, P-32 or Tm-170. The nuclide can be supported on a non-radioactive support or can be comprised in a metallic, plastic or ceramic matrix. Mixtures of these materials may also be used. Preferred are embodiments, wherein the nuclide is embedded in a matrix, most preferably metallic matrices are used. In these the nuclide may be embodied in elemental form i.e. to yield an alloy or in form of a compound such as an oxide, halogenide or carbide, nitride and so on. Corresponding suitable radiation emitting elements are e.g. disclosed in European patent application EP-A-1 084 733, the content of which is incorporated herein by way of reference.

The shielding section (3) may comprise and preferably consist of a metallic, ceramic or plastic material, preferably a metallic material, most preferably a metallic material selected from high atomic number metals. Preferably these metals are selected from the group consisting of Pt, Pd, Au, Ag, Ir, Pb, W and their alloys, compounds, and composites and mixtures thereof. Especially, carbides and nitrides and composite materials of the same can be used.

The means for containment (2, 2a) may comprise and preferably consist of a metallic, ceramic or plastic material. In case of metallic materials these are preferably selected from the group consisting of Al, Ag, Au, Pb, Cd, Ce, Cr, Co, Cu, Fe (especially stainless steel), Hg, Hf, Bi, In, Mg, Mn, Mo, Nb, Ni, Pd, Pt, Pr, Re, Rh, Sn, Si, Ta, Ti, Tb, Th, V, W, Y, Yb, Zn, Zr and their alloys, compounds, and composites and mixtures thereof. Carbides and nitrides of these materials may be used. Preferred are biocompatible materials such as Ti and its alloys. In case the shielding section forms an integral part of the means of containment, both are preferably made from the same material. In this case high atomic number metals as listed above are typically used.

The means for containment is preferably provided in a tubular or cylindrical form. For a sealing enclosure of the radiation emitting element this tubular or cylindrical form may be provided with endcaps or endplugs on both sides thereof. Similarly the radiation emitting element (1) according to the invention may be provided in tubular or cylindrical form (hollow and solid), preferably with a diameter such as to closely fit into the internal diameter of the means for containment. The radiation emitting element may, however, also be comprised of one or more spherical elements (1a). these are typically spheres of a diameter closely matching the internal diameter of the means for containment, although smaller spheres may likewise be used.

The radiation source of the present invention may further comprise a cover sleeve provided around the means for containment. Said cover sleeve may, but needs not sealingly enclose the means for containment, though a sealing enclosure is preferred. As an example, the cover sleeve may be provided in form of a (third) cylinder around the means for containment.

The cover sleeve is made of any appropriate radiation resistant, non-corrosive, biocompatible material. Preferably it consists of any of the metallic materials listed above for the means for containment, Ti, Al, Ni, Fe (stainless steel) and their alloys being especially preferred.

Providing the cover sleeve around the means for containment bears the advantage that (1) any high Z materials which need not be biocompatible can be prevented from leaching into the body, (2) any edges of the means for containment are covered to prevent damages to surrounding tissue upon insertion and removal, and (3) the source can be easily cleaned and sterilized for re-use.

Like the means for containment, the cover sleeve may also be attached to or from part of the applicator tip, e. g. as a hollow cylindrical extension thereof, receiving the means for containment which is sealed therein by an endplug. Endplugs are preferably attached by laser welding.

By providing the means for containment of the radiation source of the present invention with a radiation shielding section (3) and a radiation transition section (4), the radiation source of the present invention allows, differing from prior art radiation sources, which typically show axially homogenous radiation patterns (i.e. a pattern of homogenous irradiation around its circumference), an intentionally inhomogeneous radiation pattern. More precisely, the emitted radiation is shielded at least on one and preferably to but one side of source. Accordingly, undue radiation of healthy tissue opposing and surrounding the site to be treated can be prevented as the emitted radiation is directed appropriately. This is especially important in ophthalmic applications such as irradiation of the macula in case of macula degeneration. In these cases the radiation source needs to be placed in between macula and retina, irradiation of the retina being highly undesirably.

The radiation source of the present invention allows the precise irradiation of the involved spot of the macula without irradiating the retina in parallel. Further, by an appropriate adaption of the radiation transition area (4) the source of the present invention allows a substantial homogenous irradiation of the macular part to be treated. In addition, this part to be treated can be substantial larger than the diameter and length of the radiation source itself. Accordingly, smaller sources can be used for treating larger patches of macula, which advantageously reduces the size of inscissions and invasions needed for a successful treatment. This in turn of course reduces risks incurred by the patient during the treatment/surgery.

The present invention will be illustrated by the following example, which is not intended to limit the same.

### Example

A radiation source was manufactured by providing a wire, comprising an oxide of yttrium-90 dispersed within a metallic matrix, in this case an aluminum matrix. The wire was inserted in a first metallic cylinder, sealed by endpugs, as the means for containment. The first metallic cylinder was made of stainless steel, the endplugs being made from the same material. The first sealed metallic cylinder was inserted in a second cylinder made from an Pt/Ir-alloy, which second cylinder was provided around said first cylinder with closely matching dimensions to firmly hold said first sealed cylinder.

The first halfcircle of said second cylinder around the means for containment and aligned with the cylinder axis thereof forms the shielding section. The second halfcircle around the means for containment and aligned with the cylinder axis thereof forms the radiation transition section by varying the thickness of the Pt/Ir-alloy along the length of the second cylinder.

Eight recesses (7) are provided in a regular pattern along a central crossbridge along the longitudinal axis of the second cylinder. These recesses form windows, i. e. openings allowing access to the first cylinder below.

The source of this example further comprises a cover sleeve in form of an end plugged, thin-walled, third cylinder provided around the second cylinder to receive and hold the same. The cover sleeve is made from Ti or a Ti-alloy. It seals the radiation source and provides a cover for any edges of the second cylinder. All cylinders are affixed to each other and/or sealed by laser welding.

The emitted radiation field of the source was tested in a target area of about 4-6 mm diameter at a distance of about 1-2 mm from the source surface. The field was homogeneous within limits of about 30 % variation in dose from center to fringe. In comparison, a source not having a transition section according to the invention showed deviations of more than 50 % from center to fringe.

Although having been described with respect to preferred embodiment as set out above, this description is not to be considered limiting in that the skilled worker will appreciate the possibility of several variations of the invention as defined in the appending claims without departing from its scope.

## Claims

1. Radioactive radiation source for brachytherapy having an elongated radiation emitting element (1) within an elongated means for containment (2) such that the longitudinal axis of the radiation emitting element and the longitudinal axis of said means for containment are aligned, said means for containment comprising a shielding section (3) and a radiation transition section (4),
- said shielding section (3) covering said radiation emitting element at least partially to substantially attenuate any radiation emitted in the direction of said shielding section, **characterized in that**
- said radiation transition section (4) extends substantially along the longitudinal axis of the means for containment and comprising a shielding material (5),
- which shielding material (5) is so adapted as to attenuate the radiation emitted from said radiation emitting element such that, in a plane at a pre-selected distance from the radiation source, a substantially uniform radiation dose is received over a target area (6) having a length substantially larger than the longitudinal dimension of the elongated means for containment, and preferably also a diameter substantially larger than the diameter of the means for containment.

2. Radiation source according to claim 1, wherein said shielding section (3) and said radiation transition section (4) form integral parts of the means for containment (2).

3. Radiation source according to claim 1, wherein said means for containment (2) comprises a first layer or capsule (2a), which layer or capsule sealingly encloses the radiation emitting element (1), and further comprises a shielding section (3) and a radiation transition section (4) which are provided separately from said first layer or capsule (2a) on the outside or inside thereof.

4. Radiation source according to claim 1, wherein said shielding material (5) is adapted by varying its thickness, density and/or composition.

5. Radiation source of claim 4, wherein the shielding material (5) is provided in a greater thickness in such portions of the radiation transition section (4) closer to the target area (6).

6. Radiation source of claim 5, wherein the shielding section (3) covers the radiation emitting element (1) over the full length of its longitudinal axis on at least one side of the element.

7. Radiation source of claim 6, wherein the radiation transition section (4) is located on the side of the radiation emitting element opposite to the shielding section (3).

8. Radiation source of claim 5 or claim 6, wherein the shielding material (5) is provided with a greater shielding capacity, preferably in a greater thickness, centrally over and along the longitudinal axis of the radiation emitting element on the part of the source intended directly to oppose the target area.

9. Radiation source according to claim 8, wherein said shielding material (5) comprises recesses (7), in particular recessed windows, on such portions of the radiation transition section (4) further from the target area (6).

10. Radiation source according to one of claims 1 to 3, wherein as said shielding material (5) is adapted by varying its shielding effect through varying its density and/or composition.

11. Radiation source of claim 10, wherein a material with a higher shielding effect is used in such portions of the radiation transition section (4) closer to the target area (6), while in such portions of the radiation transition section (4) further from the target area (6) a material with a lower shielding effect is used.

12. Radiation source according to one of the claims 1 to 11, wherein said radiation source further comprises a cover sleeve enclosing the means for containment.

13. Radiation source according to one of the claims 1 to 12, wherein the radiation emitting element is provided in tubular or cylindrical form or is comprised of one or more than one spherical elements.

14. Radiation source according to one of the claims 1 to 13, wherein the radiation emitting element is a beta-radiation emitting element which preferably comprises a nuclide having a max. particle energy of β-radiation of at least 500 keV.

15. Radiation source according to one of claims 1-13 wherein the radiation emitting element comprises a nuclide selected from the group consisting of Y-90, Sr-90/Y-90, Tm-170, P-32, Cl-36, Ce-144/Pr-144, Tb-160, Ta-182, Tl-204, Sn-123, Re-188, Ir-192 and Se-75.

16. Radiation source according to claim 14, wherein the radiation emitting element comprises Sr-90/Y-90 or Y-90 in a metallic, plastic or ceramic matrix or supported on a carrier.

17. Radiation source according to one of the claims 1 to 16, wherein the shielding section (3) comprises a metallic material selected from high Z metals, preferably selected from the group consisting of Pt, Pd, Au, Ag, Ir, Pb, W and their alloys, compounds, and composites and mixtures thereof.

18. Radiation source according to one of the claims 1 to 17, wherein the means for containment (2, 2a) comprises a metallic or plastic material, preferably selected from the group consisting of Al, Ag, Au, Pb, Cd, Ce, Cr, Co, Cu, Fe, Hg, Hf, Bi, In, Mg, Mn, Mo, Nb, Ni, Pd, Pt, Pr, Re, Rh, Sn, Si, Ta, Ti, Tb, Th, V, W, Y, Yb, Zn, Zr and their alloys, compounds, and composites and mixtures thereof.

19. Radiation source of claim 1, wherein the beta-radiation emitting element (1) is a wire comprising an oxide or salt of Sr-90/Y-90 or Y-90 within a metallic matrix; said means for containment (2) comprises a first sealed metallic cylinder (2a) made of stainless steel, Ti or an alloy comprising Ti; a second cylinder made of an Pt/Ir-alloy being provided around said first cylinder, the first halfcircle of the second cylinder around the first cyliner forming said shielding section (3); and the second halfcircle of the second cylinder around the first cylinder forming the radiation transition section (4) by varying the wall thickness of the second cylinder in the second halfcircle, and wherein a cover sleeve is provided in form of a third sealed cylinder made of stainless steel, Ti or a Ti-alloy enclosing the other components of the source.

## Patentansprüche

1. Radioaktive Strahlungsquelle für die Brachytherapie, die ein lang gestrecktes Strahlungsemissionselement (1) in einem lang gestreckten Einschlussmittel (2) besitzt, derart, dass die Längsachse des Strahlungsemissionselements und die Längsachse des Einschlussmittels aufeinander ausgerichtet sind, wobei das Einschlussmittel einen Abschirmungsabschnitt (3) und einen Strahlungsübergangsabschnitt (4) umfasst,
- wobei der Abschirmungsabschnitt (3) das Strahlungsemissionselement wenigstens teilweise abdeckt, um jegliche Strahlung, die in Richtung des Abschirmungsabschnitts emittiert wird, wesentlich zu dämpfen,
**dadurch gekennzeichnet, dass**
- der Strahlungsübergangsabschnitt (4) sich im Wesentlichen längs der Längsachse des Einschlussmittels erstreckt und ein Abschirmungsmaterial (5) enthält,
- das Abschirmungsmaterial (5) so beschaffen ist, dass es die von dem Strahlungsemissionselement emittierte Strahlung in der Weise dämpft, dass in einer Ebene in einem im Voraus gewählten Abstand von der Strahlungsquelle über einem Zielbereich (6), der eine Länge besitzt, die wesentlich größer als die Längenabmessung des lang gestreckten Einschlussmittels ist, und vorzugsweise auch einen Durchmesser besitzt, der wesentlich größer als der Durchmesser des Einschlussmittels ist, eine im Wesentlichen gleichmäßige Strahlungsdosis empfangen wird.

2. Strahlungsquelle nach Anspruch 1, bei der der Abschirmungsabschnitt (3) und der Strahlungsübergangsabschnitt (4) einteilige Abschnitte des Einschlussmittels (2) bilden.

3. Strahlungsquelle nach Anspruch 1, bei dem das Einschlussmittel (2) eine erste Schicht oder Hülle (2a) umfasst, wobei diese Schicht oder Hülle das Strahlungsemissionselement (1) dicht umschließt, und ferner einen Abschirmungsabschnitt (3) und einen Strahlungsübergangsabschnitt (4) umfasst, die getrennt von der ersten Schicht oder Hülle (2a) auf deren Außenseite oder Innenseite vorgesehen sind.

4. Strahlungsquelle nach Anspruch 1, bei der das Abschirmungsmaterial (5) durch Verändern seiner Dicke, Dichte und/oder Zusammensetzung angepasst ist.

5. Strahlungsquelle nach Anspruch 4, bei der das Abschirmungsmaterial (5) in jenen Abschnitten des Strahlungsübergangsabschnitts (4), die sich näher bei dem Zielbereich (6) befinden, mit größerer Dicke vorgesehen ist.

6. Strahlungsquelle nach Anspruch 5, bei der der Abschirmungsabschnitt (3) das Strahlungsemissionselement (1) über die gesamte Länge seiner Längsachse wenigstens auf einer Seite des Elements abdeckt.

7. Strahlungsquelle nach Anspruch 6, bei der sich der Strahlungsübergangsabschnitt (4) auf jener Seite des Strahlungsemissionselements befindet, die dem Abschirmungsabschnitt (3) gegenüber liegt.

8. Strahlungsquelle nach Anspruch 5 oder Anspruch 6, bei der das Abschirmungsmaterial (5) mittig über dem Strahlungsemissionselement und entlang seiner Längsachse auf jenem Teil der Quelle, der sich direkt gegenüber dem Zielbereich befinden soll, mit einer größeren Abschirmungskapazität, vorzugsweise mit einer größeren Dicke, versehen ist.

9. Strahlungsquelle nach Anspruch 8, bei der das Abschirmungsmaterial (5) in jenen Abschnitten des Strahlungsübergangsabschnitts (4), die sich weiter von dem Zielbereich (6) entfernt befinden, Aussparungen (7), insbesondere ausgesparte Fenster, aufweist.

10. Strahlungsquelle nach einem der Ansprüche 1 bis 3, bei der das Abschirmungsmaterial (5) durch Verändern seiner Abschirmungswirkung, indem seine Dichte und/oder Zusammensetzung geändert wird, angepasst wird.

11. Strahlungsquelle nach Anspruch 10, bei der in jenen Bereichen des Strahlungsübergangsabschnitts (4), die sich näher bei dem Zielbereich (6) befinden, ein Material mit höherer Abschirmungswirkung verwendet wird, während in jenen Abschnitten des Strahlungsübergangsabschnitts (4), die sich weiter entfernt von dem Zielbereich (6) befinden, ein Material mit einer geringeren Abschirmungswirkung verwendet wird.

12. Strahlungsquelle nach einem der Ansprüche 1 bis 11, wobei die Strahlungsquelle ferner eine Abdeckhülse umfasst, die das Einschlussmittel umschließt.

13. Strahlungsquelle nach einem der Ansprüche 1 bis 12, bei der das Strahlungsemissionselement in Röhrenform oder zylindrischer Form vorgesehen ist und ein oder mehr als ein sphärisches Element umfasst.

14. Strahlungsquelle nach einem der Ansprüche 1 bis 13, bei der das Strahlungsemissionselement ein β-Strahlungs-Emissionselement ist, das vorzugsweise ein Nuklid enthält, das eine maximale β-Strahlungs-Partikelenergie von wenigstens 500 keV besitzt.

15. Strahlungsquelle nach einem der Ansprüche 1-13, bei der das Strahlungsemissionselement ein Nuklid enthält, das aus der Gruppe ausgewählt ist, die besteht aus: Y-90, Sr-90/Y-90, Tm-170, P-32, Cl-36, Ce-144/Pr-144, Tb-160, Ta-182, Tl-204, Sn-123, Re-188, Ir-192 und Se-75.

16. Strahlungsquelle nach Anspruch 14, bei der das Strahlungsemissionselement Sr-90/Y-90 oder Y-90 in einem metallischen oder keramischen Kunststoff-Grundstoff enthält oder auf einem Träger unterstützt ist.

17. Strahlungsquelle nach einem der Ansprüche 1 bis 16, bei der der Abschirmungsabschnitt (3) ein metallisches Material enthält, das aus Metallen mit hoher Z-Zahl ausgewählt ist und vorzugsweise aus der Gruppe ausgewählt ist, die besteht aus Pt, Pd, Au, Ag, Ir, Pb, W und ihren Legierungen, Verbindungen, Verbundstoffen und Gemischen hiervon.

18. Strahlungsquelle nach einem der Ansprüche 1 bis 17, bei der das Einschlussmittel (2, 2a) ein Metall- oder Kunststoffmaterial enthält, das vorzugsweise aus der Gruppe ausgewählt ist, die besteht aus Al, Ag, Au, Pb, Cd, Ce, Cr, Co, Cu, Fe, Hg, Hf, Bi, In, Mg, Mn, Mo, Mb, Ni, Pd, Pt, Pr, Re, Rh, Sn, Si, Ta, Ti, Tb, Th, V, W, Y, Yb, Zn, Zr und ihren Legierungen, Verbindungen und Verbundstoffen und Gemischen hiervon.

19. Strahlungsquelle nach Anspruch 1, bei der das Betastrahlungsemissionselement (1) ein Draht ist, der ein Oxid oder Salz von Sr-90/Y-90 oder Y-90 in einem metallischen Grundstoff enthält; wobei das Einschlussmittel (2) einen ersten dichten Metallzylinder (2a), der aus rostfreiem Stahl, Ti oder einer Ti-haltigen Legierung hergestellt ist; und einen zweiten Zylinder, der aus einer Pt/Ir-Legierung hergestellt und um den ersten Zylinder herum vorgesehen ist, umfasst, wobei der erste Halbkreis des zweiten Zylinders um den ersten Zylinder den Abschirmungsabschnitt (3) bildet; und der zweite Halbkreis des zweiten Zylinders um den ersten Zylinder den Strahlungsübergangsabschnitt (4) bildet, indem die Wanddicke des zweiten Zylinders in dem zweiten Halbkreis verändert ist, und wobei eine Abdeckhülse in Form eines dritten dichten Zylinders vorgesehen ist, der aus rostfreiem Stahl, Ti oder einer Ti-Legierung hergestellt ist und die anderen Komponenten der Quelle umschließt.

## Revendications

1. Source radioactive de rayonnement pour la brachythérapie comprenant un élément allongé (1) d'émission de rayonnement à l'intérieur d'un moyen de logement allongé (2) de telle manière que l'axe longitudinal de l'élément d'émission de rayonnement et l'axe longitudinal dudit moyen de logement soient alignés, ledit moyen de logement comprenant une section de blindage (3) et une section de transition de rayonnement (4), ladite section de blindage (3) couvrant au moins partiellement ledit élément d'émission de rayonnement pour atténuer sensiblement tout rayonnement émis en direction de ladite section de blindage, **caractérisée en ce que** ladite section de transition de rayonnement (4) s'étend sensiblement le long de l'axe longitudinal du moyen de logement et comprend une matière de blindage (5), matière de blindage (5) qui est adaptée de façon à atténuer le rayonnement émis par ledit élément d'émission de rayonnement de telle manière que, dans un plan situé à une distance présélectionnée de la source de rayonnement, une dose sensiblement uniforme de rayonnement soit reçue sur une zone cible (6) présentant une longueur sensiblement plus grande que la dimension longitudinale du moyen de logement allongé, et de préférence aussi un diamètre sensiblement plus grand que le diamètre du moyen de logement.

2. Source de rayonnement selon la revendication 1, dans laquelle ladite section de blindage (3) et ladite section de transition de rayonnement (4) font partie intégrante du moyen de logement (2).

3. Source de rayonnement selon la revendication 1, dans lequel ledit moyen de logement (2) comprend une première couche ou capsule (2a), couche ou capsule qui entoure de façon étanche l'élément d'émission de rayonnement (1), et comprend en outre une section de blindage (3) et une section de transition de rayonnement (4), qui sont formées séparément de ladite première couche ou capsule (2a) sur le coté extérieur ou le côté intérieur de celle-ci.

4. Source de rayonnement selon la revendication 1, dans laquelle ladite matière de blindage (5) est adaptée en faisant varier son épaisseur, sa densité et/ou sa composition.

5. Source de rayonnement selon la revendication 4, dans laquelle la matière de blindage (5) est fournie avec une plus forte épaisseur dans les parties de la section de transition de rayonnement (4) plus proches de la zone cible (6).

6. Source de rayonnement selon la revendication 5, dans laquelle la section de blindage (3) couvre l'élément d'émission de rayonnement (1) sur toute la longueur de son axe longitudinal sur au moins un côté de l'élément.

7. Source de rayonnement selon la revendication 6, dans laquelle la section de transition de rayonnement (4) est située sur le côte de l'élément d'émission de rayonnement opposé à la section de blindage (3).

8. Source de rayonnement selon la revendication 5 ou la revendication 6, dans laquelle la matière de blindage (5) est fournie avec une plus grande capacité de blindage, de préférence avec une plus forte épaisseur, au centre au-dessus et le long de l'axe longitudinal de l'élément d'émission de rayonnement sur la partie de la source destinée à faire face directement à la zone cible.

9. Source de rayonnement selon la revendication 8, dans laquelle ladite matière de blindage (5) comprend des cavités (7), en particulier des fenêtres en retrait, sur les parties de la section de transition de rayonnement (4) plus éloignées de la zone cible (6).

10. Source de rayonnement selon l'une quelconque des revendications 1 à 3, dans laquelle ladite matière de blindage (5) est adaptée en faisant varier son effet de blindage en faisant varier sa densité et/ou sa composition.

11. Source de rayonnement selon la revendication 10, dans laquelle on utilise une matière avec un effet de blindage plus élevé dans les parties de la section de transition de rayonnement (4) plus proches de la zone cible (6), tandis que l'on utilise une matière avec un effet de blindage plus faible dans les parties de la section de transition de rayonnement (4) plus éloignées de la zone cible (6).

12. Source de rayonnement selon l'une quelconque des revendications 1 à 11, dans laquelle ladite source de rayonnement comprend en outre un manchon de recouvrement entourant le moyen de logement.

13. Source de rayonnement selon l'une quelconque des revendications 1 à 12, dans laquelle l'élément d'émission de rayonnement est fourni sous forme tubulaire ou cylindrique ou est composé d'un ou de plus d'un élément sphérique.

14. Source de rayonnement selon l'une quelconque des revendications 1 à 13, dans laquelle l'élément d'émission de rayonnement est un élément d'émission de rayonnement bêta qui comprend de préférence un nucléide présentant une énergie de particule maximale du rayonnement β d'au moins 500 keV.

15. Source de rayonnement selon l'une quelconque des revendications 1 à 13, dans laquelle l'élément d'émission de rayonnement comprend un nucléide sélectionné dans le groupe composé de Y-90, Sr-90/Y-90, Tm-170, P-32, Cl-36, Ce-144/Pr-144, Tb-160, Ta-182, Tl-204, Sn-123, Re-188, Ir-192 et Se-75.

16. Source de rayonnement selon la revendication 14, dans laquelle l'élément d'émission de rayonnement comprend du Sr-90/Y-90 ou du Y-90 dans une matrice métallique, plastique ou céramique ou supporté sur un porteur.

17. Source de rayonnement selon l'une quelconque des revendications 1 à 16, dans laquelle la section de blindage (3) comprend une matière métallique sélectionnée parmi les métaux à haut Z, de préférence sélectionnée dans le groupe composé de Pt, Pd, Au, Ag, Ir, Pb, W et leurs alliages, composés ou composites et mélanges de ceux-ci.

18. Source de rayonnement selon l'une quelconque des revendications 1 à 17, dans laquelle le moyen de logement (2, 2a) comprend une matière métallique ou plastique, de préférence sélectionnée dans le groupe composé de Al, Ag, Au, Pb, Cd, Ce, Cr, Co, Cu, Fe, Hg, Hf, Bi, In, Mg, Mn, Mo, Nb, Ni, Pd, Pt, Pr, Re, Rh, Sn, Si, Ta, Ti, Tb, Th, V, W, Y, Yb, Zn, Zr et leurs alliages, composés, et composites et mélanges de ceux-ci.

19. Source de rayonnement selon la revendication 1, dans laquelle l'élément d'émission de rayonnement bêta (1) est un fil comprenant un oxyde ou un sel de Sr-90/Y-90 ou de Y-90 à l'intérieur d'une matrice métallique; ledit moyen de logement (2) comprend un premier cylindre métallique scellé (2a) constitué d'acier inoxydable, de Ti ou d'un alliage contenant du TI; un deuxième cylindre constitué d'un alliage Pt/Ir étant prévu autour dudit premier cylindre, le premier demi-cercle du deuxième cylindre autour du premier cylindre formant ladite section de blindage (3); et le second demi-cercle du deuxième cylindre autour du premier cylindre formant la section de transition de rayonnement (4) en faisant varier l'épaisseur de paroi du deuxième cylindre dans le second demi-cercle, et dans laquelle il est prévu un manchon de recouvrement sous la forme d'un troisième cylindre scellé constitué d'acier inoxydable, de Ti ou d'un alliage de Ti entourant les autres composants de la source.
